Europäisches Patentamt

European Patent Office

Office européen des brevets

(19)

(11) Veröffentlichungsnummer: **0 259 583 B1**

(12) **EUROPÄISCHE PATENTSCHRIFT**

(45) Veröffentlichungstag der Patentschrift: 13.02.91

(21) Anmeldenummer: 87110576.3

(22) Anmeldetag: 22.07.87

(51) Int. Cl.⁵: **C02F 1/26**, C07C 51/48, C07C 37/72, C07C 45/80, C07C 29/86, C07C 47/058

(54) **Kontinuierliches Verfahren zur Extraktion von Carbonsäuren, Aldehyden, Ketonen, Alkoholen und Phenolen aus verdünnten wässerigen Lösungen.**

(30) Priorität: 08.08.86 DE 3626968

(43) Veröffentlichungstag der Anmeldung:
16.03.88 Patentblatt 88/11

(45) Bekanntmachung des Hinweises auf die
Patenterteilung:
13.02.91 Patentblatt 91/07

(84) Benannte Vertragsstaaten:
AT BE CH DE FR GB IT LI NL SE

(56) Entgegenhaltungen:
EP-A- 0 036 406
EP-A- 0 105 161
EP-A- 0 156 597
US-A- 439 900
US-A- 4 544 779

(73) Patentinhaber: HOECHST AKTIENGESELL-
SCHAFT
Postfach 80 03 20
D-6230 Frankfurt am Main 80(DE)

(72) Erfinder: Weferling, Norbert,Dr.
Schaesbergstrasse 13
D-5030 Hürth(DE)
Erfinder: Pfüller, Peter, Dr.
Samerbergweg 9
D-8269 Burgkirchen(DE)
Erfinder: Kanzler, Walter,
Stiftingtalstrasse 165 d
A-8010 Graz(AT)
Erfinder: Schedler, Johannes
Mittergrabenweg 72
A-8010 Graz(AT)
Erfinder: Thalhammer, Heimo, Dr.
Wiesenhofweg 9
A-8071 Dörfla(AT)

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein kontinuierliches Verfahren zur Extraktion von Carbonsäuren, Aldehyden, Ketonen, Alkoholen und Phenolen, insbesondere von Carbonsäuren mit einer Kohlenstoffkette von 1 - 7 C-Atomen, Benzoesäure, Maleinsäure, Phthalsäure sowie Furfural, aus verdünnten wässerigen Lösungen mit einem mit Wasser nicht mischbaren, flüssigen Extraktionsmittel, gegebenenfalls zusammen mit einem wasserunlöslichen Lösemittel.

Bei verschiedenen chemischen Produktionen, wie beispielsweise dem Aufschluß von Pflanzenmaterial oder dessen Abfällen in Zellstoffabriken, entstehen mit organischen Bestandteilen stark verunreinigte Abwässer, die für die biologische Abwasseraufbereitung große Probleme darstellen. Je nach der Art des technischen Prozesses entstehen beispielsweise neben Mono-, Oligo- und Polysacchariden größere Mengen Carbonsäuren, Aldehyde, Ketone, Alkohole oder Furfural.

Eine Möglichkeit zur Vorreinigung dieser Abwässer besteht darin, das Abwasser einer Destillation zuzuführen und die dabei anfallenden Rückstände zu verbrennen. Dieses Verfahren hat einen hohen Energiebedarf und ist daher unwirtschaftlich. Es hat sich weiter gezeigt, daß die im Abwasser vorhandenen organischen Bestandteile zusammen mit den Brüden kondensiert werden und somit über den Vorfluter die biologische Abwasseraufbereitung belasten.

In der Technik ist man daher dazu übergegangen, die organischen Bestandteile mittels eines Extraktionsvorganges aus dem Abwasser zu eliminieren. Dieses Verfahren hat den Vorteil, daß die extrahierten Bestandteile als technisch nutzbare Verbindungen erhalten werden können.

In der DE-24 23 272 C2 wird ein Verfahren zur extraktiven Abtrennung von Carbonsäuren mit einem mit Wasser nicht mischbaren, flüssigen Extraktionsmittel vorgeschlagen, in welchem zusätzlich spezielle Dialkylalkylphosphonate, Alkyldialkylphosphinate oder Trialkylphosphanoxid dem Extraktionsmittel zugesetzt sind.

In der US-PS 3 816 524 wird ein Verfahren zur Extraktion von Carbonsäuren vorgeschlagen, bei dem ebenfalls Phosphanoxide, beispielsweise Trioctylphosphanoxid, dem flüssigen, organischen Extraktionsmittel zugesetzt sind.

Nachteilig bei diesen Verfahren ist der geringe Verteilungskoeffizient, d.h. es müssen große Mengen an Extraktionsmitteln gehandhabt werden und es verbleibt noch ein relativ großer Restgehalt von organischen Verbindungen im Abwasser, sowie der hohe Energiebedarf bei der Aufarbeitung des beladenen Extraktionsmittels bei der Destillation und der hohe Schmelzpunkt des Trioctylphosphanoxids, wodurch eine Extraktion bei tieferen Temperaturen (etwa 40 °C) wegen der Ausscheidung einer festen Phase ausgeschlossen wird.

Zur Beseitigung eines Teiles der Mängel ist deshalb in der EP 0 132 700 AI vorgeschlagen worden, eine Mischung von 2 oder mehreren verschiedenen Phosphanoxiden als Extraktionsmittel zu nutzen. Bei dem Gemisch aus Tri-n-octylphosphanoxid mit Tri-n-hexylphosphanoxid kann die Extraktion auch bei Temperaturen unterhalb von 40 °C durchgeführt werden, da dieses Extraktionsmittelgemisch bei Temperaturen unterhalb 40 °C als flüssige Phase vorliegt.

Die Abwasser-Extraktion bei tiefen Temperaturen ist wünschenswert, da der Verteilungskoeffizient für tiefere Temperaturen günstigere Werte hat.

Bei der Verwendung von Phosphanoxid-Gemischen als Extraktionsmittelgemisch hat sich die schwierige Aufarbeitung des Extraktionsmittelgemisches herausgestellt. Wegen der ca. 10 mal so hohen Löslichkeit von Tri-n-hexylphosphanoxid gegenüber Tri-n-octylphosphanoxid kommt es zu größeren Tri-n-hexylphoshanoxid-Verlusten, die im extrahierten Abwasser verbleiben, so daß sich die prozentuale Zusammensetzung des Phosphanoxid-Gemisches während der kontinuierlich betriebenen Extraktion verändert und durch weitere Maßnahmen überwacht und neu eingestellt werden muß.

Es war die Aufgabe gestellt, ein unter Normalbedingungen flüssiges Extraktionsmittel anzugeben, welches eine chemisch einheitliche Verbindung ist, die ferner eine geringe Wasserlöslichkeit hat und einen hohen Verteilungskoeffizienten für die Extraktion von oxidischen organischen Verbindungen aus verdünnten wässerigen Lösungen aufweist.

Es wurde nunmehr gefunden, daß ein kontinuierliches Verfahren zur Extraktion von Carbonsäuren, Aldehyden, Ketonen, Alkoholen und Phenolen, insbesondere von Carbonsäuren mit einer Kohlenstoffkette von 1 - 7 C-Atomen, Benzoesäure, Maleinsäure, Phthalsäure sowie Furfural, aus verdünnten wässerigen Lösungen mit einem mit Wasser nicht mischbaren, flüssigen Extraktionsmittel, gegebenenfalls zusammen mit einem wasserunlöslichen Lösemittel, betrieben werden kann, wenn als Extraktionsmittel s-Butyl-di-n-octylphosphanoxid verwendet wird.

Als Lösemittel können Alkane mit 6 - 13 C-Atomen oder Aromaten mit einem Siedepunkt zwischen 130 und 250 °C, insbesondere Kerosin oder n-Undecan, verwendet werden.

Das erfindungsgemäße Verfahren ist insbesondere geeignet, wenn die verdünnten wässerigen Lösungen Abwässer aus chemischen Produktionen sind, insbesondere solche, die beim Aufschluß von Pflanzenmaterial oder dessen Abfällen in Zellstoffabriken anfallen.

Es kann zweckmäßig sein, wenn ein s-Butyl-di-n-octylphosphanoxid-Lösemittel-Gemisch mit einem Lösemittelgehalt bis zu 80 Gew% verwendet wird.

Vorzugsweise wird die Extraktion bei einer Temperatur von 15 bis 80°C, insbesondere 30 bis 60°C, durchgeführt und im Gegenstrom in einem mehrstufigen Extraktor geführt, wobei das Volumen-Verhältnis wässerige Lösung : s-Butyl-di-n-octylphosphanoxid 1 bis 10, insbesondere 2 bis 4, beträgt.

Das erfindungsgemäße Verfahren kann auch in einem Rührbehälter erfolgen, wobei dann ein Volumen-Verhältnis wässerige Lösung : s-Butyl-di-n-octylphosphanoxid von 0,5 bis 5 : 1, insbesondere 1 bis 2 : 1, eingehalten wird.

Vorzugsweise erfolgt die Aufarbeitung des Extraktes auf destillativem Wege.

Es hat sich gezeigt, daß es sinnvoll sein kann, wenn die Isolierung von Carbonsäuren und Phenolen aus dem Extrakt mit Alkali unter Salzbildung erfolgt.

Die Erfindung wird anhand der Beispiele erläutert.

Herstellung von s-Butyl-di-n-octylphosphanoxid

a) Herstellung von sek-Butylphosphan

In einem 90 l-Autoklaven wurden 4,8 kg (141,2 Mol) $PH_3$ einkondensiert und anschließend auf 80°C aufgeheizt. über eine Membrandosierpumpe wurde eine unter einem Druck von 6 bar stehende Lösung von 410 g (2,1 Mol) 2,2'Azobis-(2-methyl-butyronitril) (VAZO 67®, DuPont) in 7,9 kg (141 Mol) Buten-2 innerhalb von 1 Std. zudosiert. Die Raktionstemperatur stieg auf 90°C, während der Druck kontinuierlich von 84 bar auf 38 bar sank. Man ließ noch 1 Std. bei 90°C nachreagieren, entspannte den Reaktor nach Abkühlung auf Raumtemperatur, spülte den Reaktionsraum mit Stickstoff und überführte das Produkt in ein Destillationsgefäß. Zur Gewinnung größerer Mengen wiederholte man diese Reaktion achtmal, bevor das gesammelte Rohprodukt destillativ aufgearbeitet wurde. Insgesamt wurden 45,8 kg sek-Butylphosphan, Kp 72 - 74°C (1013 mbar); $^{31}$P-NMR : - 115 ppm, > 99 Mol % (Ausbeute: 46 %, bez. auf eingesetztes $PH_3$) neben 3,9 kg Di-sek-butylphosphan, Kp 154 - 158°C (1013 mbar); $^{31}$P-NMR : - 23,6, - 28,6, - 33,9 ppm; Intensitäten 1 : 2 : 1, > 99 Mol % erhalten.

b) Herstellung von sek-Butyldioctylphosphan

Eine nach dem oben beschriebenen Verfahren hergestellte Phosphanmenge von 54,9 kg (610 Mol) wurde in einem 800 l-Rührkessel vorgelegt und auf 80°C aufgeheizt. Innerhalb von 2 Std. wurde eine Lösung von 5,9 kg (30,5 Mol) VAZO 67® in 532 kg (4,74 kMol) Octen-1 dazugepumpt. Man ließ 8 Std. bei 80 - 85°C reagieren und trennte danach die Leichtsieder (überschüssiges Octen, nicht vollständig abreagierte Phosphorprodukte) im Vak. (1 mbar) bis zu einer Sumpftemperatur von 155°C ab. Es wurden 183 kg sek-Butyldioctylphosphan (96 %, bez. auf eingesetztes Phosphan) in Form einer leicht gelblichen, geruchlosen Flüssigkeit erhalten. $^{31}$P-NMR: - 19,3 ppm, > 98 Mol %.

c) sek-Butyldioctylphosphanoxid

In einem 300 l-Emaille-Rührbehälter wurden gleichzeitig Butyldioctylphosphan (Volumenstrom: 88 l/h) und 35 %ige Wasserstoffperoxidlösung (Volumenstrom: 21 l/h) eindosiert und bei 45 bis 55°C unter Kühlung zur Reaktion gebracht. Insgesamt wurden 181 kg tert. Phosphan (576 Mol) und 72 kg $H_2O_2$ 35 %ig (605 Mol) eingesetzt. Man ließ 1 Std. bei 60°C nachreagieren und dosierte dann unter Kontrolle des pH-Wertes der Reaktionsmischung eine 20 %ige Lösung von Soda (Dekahydrat) in Wasser zu. Bei einem pH-Wert von 8,5 ≙ 120 ml Lösung wurde die Zugabe gestoppt. Man ließ noch 1 Std. bei 60°C rühren und schaltete dann den Rührer ab. Innerhalb von 30 Min trennte sich die Emulsion in eine untere wässerige und eine obere Phosphanoxid-Phase. Das Wasser wurde abgetrennt und verworfen. Den Rest überführte man in eine Destillationsblase und trieb restliches Wasser bei einem Vakuum von 1 mbar und einer Sumpftemperatur von 160°C ab. Das gestrippte sek-Butyldioctylphosphanoxid wurde bei Raumtemperatur als leicht gelbliche ölige Flüssigkeit abgefüllt. $^{31}$P-NMR: + 51,1 ppm, > 97 Mol %; Ausbeute: 186 kg (98 %, bez. auf eingesetztes Phosphan).

Beispiel 1

5 m$^3$/h Abwasser eines Phenol-verarbeitenden Betriebes, mit einem Gehalt von 5 Gew% Phenol werden mit 1,3 m$^3$/h s-Butyl-di-n-octylphosphanoxid bei einer Temperatur von 30°C in einem Rührzellenextraktor mit 3,5 theoretischen Trennstufen im Gegenstrom extrahiert.

Die abgelassene wässerige Phase enthält 11 ppm Phenol. Das beladene Extraktionsmittel enthält neben 17 Gew% Phenol noch 0,5 Gew% Wasser, das in einem Verdampfer bei 140°C unter Normaldruck gestrippt wird. Die gestrippten 7 kg/h Wasser werden in den Rührzellenextraktor rückgeführt.

In einer nachgeschalteten Kolonne wurde der Extrakt bei einem Unterdruck von 0,05 bar und einer Kopftemperatur von 95° C getrennt. Aus dem Abscheider werden 250 kg/h Phenol abgezogen.

Der Blaseninhalt, das s-Butyl-di-n-octylphosphanoxid wurde als Extraktionsmittel in den Rührzellenextraktor rückgeführt.

Beispiel 2

100 m³/h Brüdenkondensat aus der Zellstoffproduktion, enthaltend 1,5 Gew% Essigsäure, 0,1 Gew% Ameisensäure, 0,1 Gew% Methanol und 0,4 Gew% Furfural werden in einem Rührzellenextraktor mit 3,5 theoretischen Trennstufen im Gegenstrom mit 100 m³/h Extraktionsmittel, bestehend aus 36,4 Volumen% s-Butyl-di-n-octylphosphanoxid, Rest n-Undecan, bei 40° C extrahiert.

Das extrahierte Brüdenkondensat enthält noch

| 105 kg/h | Essigsäure |
| 8 kg/h | Ameisensäure |
| 12 kg/h | Furfural |
| 50 kg/h | Methanol |

Der Extrakt wird in einem Verdampfer gestrippt und als Kopfprodukt 1,6 m³/h Wasser abgezogen, das in den Rührzellenextraktor rückgeführt wird.

In einer nachgeschalteten Kolonne wird der Extrakt bei einem Unterdruck von etwa 0,2 bar und 140° C Kopftemperatur getrennt. Aus der Blase wird das s-Butyl-di-n-octylphosphanoxid/n-Undecan-Gemisch als Extraktionsmittel in den Rührzellenextraktor rückgeführt.

Aus dem Abscheider der Kolonne werden 1,9 m³/h, enthaltend die aus dem Brüdenkondensat extrahierten organischen Verbindungen, zusammen mit 0,2 m³/h Wasser einer weiteren destillativen Trennung in die Einzelkomponenten unterworfen. Diese Trennung kann beispielsweise gemäß der in der AT-PS 365 080 beschriebenen Arbeitsweise erfolgen.

Nach der destillativen Trennung werden

| 1395 kg/h | Essigsäure |
| 92 kg/h | Ameisensäure |
| 388 kg/h | Furfural |
| 50 kg/h | Methanol |

erhalten.

Beispiel 3

100 m³/h Abwasser aus einer Fischer-Tropsch-Synthese-Anlage, enthaltend

| 0,866 Gew% | Essigsäure |
| 0,242 Gew% | Propionsäure |
| 0,110 Gew% | Buttersäure |
| 0,035 Gew% | Valeriansäure |
| 0,004 Gew% | Methanol, |

werden in einem Rührzellenextraktor mit 3,5 theoretischen Trennstufen im Gegenstrom mit 80 m³/h eines Gemisches, bestehend aus 40 Volumen% s-Butyl-di-n-octylphosphanoxid und 60 Volumen% n-Dodecan, bei 30° C extrahiert.

Mit dem extrahierten Abwasser werden

| 16 kg/h | Essigsäure |
| 12 kg/h | Propionsäure |
| 1 kg/h | Buttersäure |
| 1 kg/h | Valeriansäure |
| 2 kg/h | Methanol |

in den Vorfluter abgelassen.

Der Extrakt aus dem Rührzellenextraktor wird in einem Verdampfer gestrippt. Als Kopfprodukt werden 1,28 m³/h Wasser abgezogen, die zum Rührzellenextraktor rückgeführt werden.

In einer nachgeschaltenen Kolonne wird der Extrakt bei einem Unterdruck von etwa 0,2 bar und 135° C Kopftemperatur destillativ getrennt.

Aus der Blase wird das s-Butyl-di-n-octylphosphanoxid/n-Dodecan-Gemisch als Extraktionsmittel in den Rührzellenextraktor rückgeführt.

Aus dem Abscheider der Kolonne werden 1,2 m³/h, enthaltend die extrahierten organischen Verbindungen aus dem Abwasser, und 0,15 m³/h Wasser einer weiteren destillativen Trennung in die Einzelkomponenten unterworfen.

Aus der Destillation werden

| 850 kg/h | Essigsäure |
| 230 kg/h | Propionsäure |
| 109 kg/h | Buttersäure |
| 34 kg/h | Valeriansäure |
| 2 kg/h | Methanol |

erhalten.

Vergleichsbeispiel

Es wird das erfindungsgemäße Verfahren mit einem Extraktionsmittelgemisch, bestehend aus 40 Volumen% eines Gemisches, enthaltend 65 Gew% Tri-n-hexylphosphanoxid und 35 Gew% Tri-n-octylphosphanoxid, und 60 Volumen% n-Dodecan verglichen, entsprechend der Arbeitsweise der EP-0 132 700 Al. Dazu werden 100 m³/h Abwasser einer Fischer-Tropsch-Synthese-Anlage mit 100 m³/h des o.g. Extraktionsmittelgemischs, wie im Beispiel 3 beschrieben, behandelt.

Die eingesetzte Extraktionsmittel-Menge mußte auf 100 m³/h erhöht werden, um ein Abwasser vergleichbarer Qualität zu erhalten. In der nachgeschalteten Kolonne, zur destillativen Trennung in den organischen Extrakt und das Extraktionsmittel, mußten 9 t/h Dampf aufgewendet werden, während gemäß der Arbeitsweise des Beispiels 3 nur 5,8 t/h Dampf benötigt wurden.

## Ansprüche

1. Kontinuierliches Verfahren zur Extraktion von Carbonsäuren, Aldehyden, Ketonen, Alkoholen und Phenolen, insbesondere von Carbonsäuren mit einer Kohlenstoffkette von 1 - 7 C-Atomen, Benzoesäure, Maleinsäure, Phthalsäure sowie Furfural, aus verdünnten wässerigen Lösungen mit einem mit Wasser nicht mischbaren, flüssigen Extraktionsmittel, gegebenenfalls zusammen mit einem wasserunlöslichen Lösemittel, dadurch gekennzeichnet, daß als Extraktionsmittel s-Butyl-di-n-octylphosphanoxid verwendet wird.

2. Verfahren nach Anspruch 1, dadurch gekennzeichnet, daß als Lösemittel Alkane mit $C_6$ - $C_{13}$ oder Aromaten mit Siedepunkten zwischen 130 und 250° C, insbesondere Kerosin oder n-Undecan, verwendet werden.

3. Verfahren nach Anspruch 1 oder 2, dadurch gekennzeichnet, daß ein s-Butyl-di-n-octyl-phosphanoxid-Lösemittel-Gemisch mit einem Lösemittelgehalt bis zu 80 Gew% verwendet wird.

4. Verfahren nach einem der Ansprüche 1 - 3, dadurch gekennzeichnet, daß die verdünnten wässerigen Lösungen die Abwässer aus chemischen Produktionen sind, insbesondere solche, die beim Aufschluß von Pflanzenmaterial oder dessen Abfällen in Zellstoffabriken anfallen.

5. Verfahren nach einem der Ansprüche 1 - 4, dadurch gekennzeichnet, daß die Extraktion bei einer Temperatur von 15 bis 80° C, insbesondere 30 bis 60° C, durchgeführt wird.

6. Verfahren nach einem der Ansprüche 1 - 5, dadurch gekennzeichnet, daß die Extraktion im Gegenstrom in einem mehrstufigen Extraktor durchgeführt wird, wobei das Volumen-Verhältnis wässerige Lösung : s-Butyl-di-n-octylphosphanoxid 1 - 10 : 1, insbesondere 2 bis 4 : 1, beträgt.

7. Verfahren nach einem der Ansprüche 1 - 5, dadurch gekennzeichnet, daß die Extraktion in einem Rührbehälter erfolgt und das Volumen-Verhältnis wässerige Lösung : s-Butyl-di-n-octylphosphanoxid 0,5 bis 5 : 1, insbesondere 1 bis 2 : 1, beträgt.

8. Verfahren nach einem der Ansprüche 1 - 7, dadurch gekennzeichnet, daß die Aufarbeitung des Extraktes auf destillativem Weg erfolgt.

9. Verfahren nach einem der Ansprüche 1 - 7, dadurch gekennzeichnet, daß die Isolierung der Carbonsäuren und Phenole aus dem Extrakt mit Alkali unter Salzbildung erfolgt.

## Claims

1. A continuous process for the extraction of carboxylic acids, aldehydes, ketones, alcohols and phenols, in particular of carboxylic acids having a carbon chain of 1 - 7 carbon atoms, benzoic acid, maleic acid, phthalic acid, and furfural from dilute aqueous solutions by means of a water-immiscible liquid extractant, if appropriate in combination with a water-insoluble solvent, which comprises using s-butyl-di-n-octylphosphane oxide as extractant.

2. The process as claimed in claim 1, wherein $C_6$ - $C_{13}$-alkanes or aromatics having boiling points between 130 and 250° C, in particular kerosene or n-undecane, are used as solvent.

3. The process as claimed in claim 1 or 2, wherein an s-butyl-di-n-octyl-phosphane oxide solvent mixture having a solvent content of up to 80 % by weight is used.

4. The process as claimed in any of claims 1 - 3, wherein the dilute aqueous solutions are the waste waters from chemical productions, in particular those formed in the digestion of plant material, or waste products thereof in pulp mills.

5. The process as claimed in any of claims 1 - 4, wherein the extraction is carried out at a temperature of 15 to 80° C, in particular 30 to 60° C.

6. The process as claimed in any of claims 1 - 5, wherein the extraction is carried out in countercurrent in a multi-step extractor, in which the volume ratio of the aqueous solution to s-butyl-di-n-octylphosphane oxide is 1 - 10 : 1, in particular 2 to 4 : 1.

7. The process as claimed in any of claims 1 - 5, wherein the extraction is carried out in a stirred vessel and the volume ratio of aqueous solution to s-butyl-din-octylphosphane oxide is 0.5 to 5 : 1, in particular 1 to 2 : 1.

8. The process as claimed in any of claims 1 - 7, wherein the extract is worked up by distillation.

9. The process as claimed in any of claims 1 - 7, wherein the carboxylic acids and phenols are isolated from the extract by means of alkali with the formation of a salt.

**Revendications**

1. Procédé continu pour l'extraction d'acides carboxyliques, d'aldéhydes, de cétones, d'alcools et de phénols, en particulier d'acides carboxyliques à chaîne carbonée en $C_1$-$C_7$, d'acide benzoïque, d'acide maléique, d'acide phtalique ainsi que de furfural, de solutions aqueuses diluées par un agent d'extraction liquide non miscible avec l'eau, éventuellement avec un solvant insoluble dans l'eau, Caractérisé en ce que l'on utilise comme agent d'extraction l'oxyde de s-butyl-di-n-octylphosphine.

2. Procédé selon la revendication 1, caractérisé en ce que l'on utilise comme solvants des alcanes en $C_6$-$C_{13}$ ou des carbures aromatiques ayant des points d'ébullition entre 130 et $250^\circ$ C, en particulier le kérosène ou le n-undécane.

3. Procédé selon la revendication 1 ou 2, caractérisé en ce que l'on utilise un mélange oxyde de s-butyl-di-n-octylphosphine-solvant ayant une teneur en solvant allant jusqu'à 80 % en poids.

4. Procédé selon l'une des revendications 1 à 3, caractérisé en ce que les solutions aqueuses diluées sont les eaux résiduaires de productions chimiques, en particulier celles qui se forment dans l'attaque de matière végétale ou de ses déchets dans les fabriques de cellulose.

5. Procédé selon l'une des revendications 1 à 4, caractérisé en ce que l'extraction est mise en oeuvre à une température de 15 à $800^\circ$ C, en particulier de 30 à $60^\circ$ C.

6. Procédé selon l'une des revendications 1 à 5, caractérisé en ce que l'extraction est mise en oeuvre à contre-courant dans un extracteur à plusieurs étages, le rapport en volume solution aqueuse : oxyde de s-butyl-di-n-octylphosphine étant de 1 - 10 : 1, en particulier de 2 à 4 : 1.

7. Procédé selon l'une des revendications 1 à 5, caractérisé en ce que l'extraction s'effectue dans un récipient à agitation et le rapport en volume solution aqueuse : oxyde de s-butyl-di-n-octylphosphine est de 0,5 à 5 : 1, en particulier de 1 à 2 :

8. Procédé selon l'une des revendications 1 à 7, caractérisé en ce que le traitement de l'extrait s'effectue par distillation.

9. Procédé selon l'une des revendications 1 à 7, caractérisé en ce que l'isolement des acides carboxyliques et des phénols de l'extrait s'effectue par un alcali avec salification.